# EUROPEAN PATENT APPLICATION

(11) **EP 4 094 721 A1**
(43) Date of publication of application: **30.11.2022**
(21) Application number: 21176297.6
(22) Date of filing: 27.05.2021
(51) Int. Cl.: A61C 17/22, A61B 5/00

(54) **ORAL SURFACE CHARACTERISTIC DETECTION**

(71) Applicant: Koninklijke Philips N.V., 5656 AG Eindhoven (NL)
(72) Inventor: BRANDAO SILVA, Priscilla, 5656 AE Eindhoven (NL); JOHNSON, Mark Thomas, 5656 AE Eindhoven (NL); GERHARDT, Lutz Christian, 5656 AE Eindhoven (NL); SPELT, Hanne Adriana Alijda, 5656 AE Eindhoven (NL); KOOIJMAN, Gerben, 5656 AE Eindhoven (NL); HIWALE, Sujitkumar, 5656 AE Eindhoven (NL)
(74) Representative: Philips Intellectual Property & Standards

(57) **Abstract**

Means for detecting oral surface characteristics, or proxies thereof, based on sensing of transient force or displacement of protruding cleaning members of an oral care device, and based on mapping said sensing data onto cleaning member position and/or motion data. From analysis of patterns in the position and/or motion data over a measurement or sampling period, surface characteristics including a measure of surface friction may be derived. In some embodiments, secondary surface characteristics may be derived based on the determined measure of surface friction.

## Description

### FIELD OF THE INVENTION

The present invention relates to the field of oral care, and in particular to the detection of surface characteristics of oral surfaces.

### BACKGROUND OF THE INVENTION

Monitoring of oral surface characteristics and modifications thereof is useful for oral health and hygiene. For example, monitoring tooth staining, tooth plaque, tooth biofilm deposits, or tooth surface wear or damage can be valuable. Other oral surfaces such as those of oral prosthetics or implants, can also be valuable.

Typically, monitoring surface characteristics must be performed manually using visual assessment. It would be advantageous to provide means for automatically monitoring tooth surface properties, for example during daily oral healthcare activities such as toothbrushing.

One common tooth surface characteristic which is monitored is tooth surface staining. Stain formation on the tooth surface occurs slowly and is difficult to detect. The detection of stains is currently performed through visual assessment. In particular, this can either be done by clinical classification of stains by determining the Lobene index or a variant thereof, or by using shade guides.

External tooth staining occurs due to accumulation of color to the tooth pellicle due to lifestyle habits, e.g. consumption of tooth-staining substances. Some persons are more predisposed to tooth staining than others. Relevant factors affecting predisposition include enamel defects, salivary dysfunction and poor oral hygiene. The latter is the most common factor, and can be managed through prophylactic actions such as regular tooth cleaning, or remedial action such as polishing, bleaching or microabrasion.

Manual methods for monitoring tooth staining require advanced training, as well as controlled illumination conditions. This technique is also difficult for areas with poor accessibility such as lingual incisors, buccal molars, or interproximal regions.

### SUMMARY OF THE INVENTION

The invention is defined by the claims.

According to examples in accordance with an aspect of the invention, there is provided a processing unit for an oral care device, adapted to: receive one or more sensor signals indicative of force exerted on, and/or deflection of, one or more flexible cleaning elements of the oral care device over a time period; obtain position and/or motion information of the one or more flexible cleaning elements over the time period based on the received one or more sensor signals; and determine one or more oral surface characteristics based on the cleaning element position and/or motion information, the one or more oral surface characteristics including a measure of surface friction.

The flexible cleaning elements may be flexible protruding cleaning elements, e.g. cleaning filaments or rods or members. The cleaning elements may for example include bristles or filaments.

The sensor signals may be obtained over, or cover, a sampling time period. The obtained position and/or motion information may be information for the sampling period. The sampling period may for example correspond to an operating session of the oral care device, e.g. a cleaning session of an oral care device.

The acquired sensor signals may be signals indicative of kinematics of the elastic cleaning elements.

The oral surface characteristics of teeth may change over time and may require time-consuming visual assessment by a trained professional that may be unreliable for hard-to-reach areas of the mouth.

It has been realized by the inventors that cleaning element kinematics or dynamics during operation of an oral care device vary in a measurable way dependent upon oral surface characteristics such as surface stain or other surface modifications. Thus, by detecting the cleaning element movement patterns during use, this provides a means for determining the surface characteristics. Where the oral surface in question is a tooth surface, the inventors have realized from experimental data that stains and/or plaque buildup on the tooth pellicle and/or wear or damage of the tooth surface may modify tooth surface characteristics. By way of example, surface roughness on a stain may be up to 10-15 times greater than surface roughness on an unstained tooth. By way of further example, the friction coefficient measured in vitro for a nylon bristle against a human saliva-black tea-based stain surface was found to be up to six times greater than a nylon bristle against an unstained tooth in the same setting. A measure of stain removal and/or wear or damage to the tooth surface may be assessed through friction measurements by monitoring the change in the (static or dynamic) friction coefficient between the cleaning element and the tooth.

The processing unit is able to use the regular interaction of cleaning elements on oral surfaces during a cleaning session to collect information about the dynamics of the cleaning elements during said interactions. From this information, it is possible to assess the oral surface characteristics without additional effort on the user's part, and in hard-to-reach areas. The oral care device may be, by way of non-limiting example, a powered toothbrush or brushing mouthpiece device.

Acquired position information may include an angular pose of the cleaning element relative to a reference axis or a reference origin, e.g. an axis normal to a support surface or body from which the cleaning element extends. Acquired position information may include position information for a distal tip of one or more of the cleaning elements within a reference co-ordinate system.

The position and/or motion information may be for each of a plurality of cleaning elements, or may be aggregate or average data pertaining to at least a subset of the plurality of cleaning elements.

The cleaning element position and/or motion information may comprise cleaning element kinematics or dynamics information such as motion patterns, motion velocity, changes in motion velocity (acceleration). The one or more surface characteristics may additionally or alternatively be based on a force pattern experienced by the cleaning element, e.g. a variation in force (in one or more of normal or shear directions) as a function of time over the signal sampling period.

The measure of oral surface friction may be a friction coefficient.

The one or more sensors may comprise any one or more of: a 3D dynamometer (e.g. piezoelectric-based); one or more strain gauges; a capacitive gauge; a Hall sensor; a fiber Bragg grating; and/or Fabry-Perot sensor.

The determined one or more surface characteristics may include one or more measures indicative of structural modifications to the oral surface, e.g. presence of a biological coating material (e.g. biofilm, dental calculus or tartar) on the surface and/or surface structural damage (in the form of tooth wear or breakage).

In some embodiments, the determining the one or more surface characteristics may comprise detecting, based on the position and/or motion information, a stick-slip condition of the oral surface.

When pulled across a surface with higher surface friction, an object will often exhibit stick-slip motion, meaning the object exhibits discontinuous motion comprising one or more transient motion periods (slip) interrupted by periods of zero motion (stick). Physically, this corresponds to the force which is pulling the object recurrently falling below the minimum force needed to overcome the static friction between the object and the surface (i.e. falling into a potential energy valley).

Stick-slip motion can be observed for cleaning elements being pulled across a tooth surface of higher surface friction. Higher surface friction has been found by the inventors to be present for stained teeth. Thus, by identifying stick-slip conditions on the tooth surface, an indication of the surface friction and/or of the presence of staining may be obtained.

In some embodiments, the one or more oral surface characteristics include any one or more of: a tooth staining level, a tooth staining color, a measure of physical wear to the tooth surface (e.g. surface erosion), presence and/or a category of bacterial biofilms, presence of dental tartar, presence of lesions in the gums or tooth, presence and status of non-biological structures such as orthodontic structures, e.g. prosthetics or implants.

For example, by determining a tooth staining level or staining color, it is possible to replace or supplement manual stain evaluation methods such as the Lobene index or a tooth shade guide. Furthermore, the determined oral surface characteristics may be easier to obtain in hard-to-reach areas of the mouth compared with visual assessment. The measure of tooth staining may be derived based on the measure of surface friction in some examples.

In some embodiments, determining the one or more oral surface characteristics may comprise accessing a database comprising data which relates cleaning element position and/or motion information to oral surface characteristics. The database may comprise one or more lookup tables. In some embodiments, determining the one or more oral surface characteristics may comprise using one or more machine learning algorithms, trained so as to receive an input comprising position and/or motion data for the one or more cleaning elements, and to generate an output comprising a measure indicative of one or more oral surface characteristics.

In some embodiments, the determining of the oral surface characteristics may comprise detecting one or more pre-defined motion signatures, or motion patterns, in the obtained sensor signals or the derived position and/or motion information. For example, certain characteristic movement patterns of the cleaning elements when engaged with teeth for a cleaning function may be associated with particular surface characteristics. In some examples, the processor may be adapted to detect properties or parameters of one or more motion signatures or patterns, for example a ratio of contact force to motion speed as a cleaning element is dragged across tooth surface, or an amplitude of an oscillatory motion.

In some embodiments, the processing unit may be adapted to generate a data representation of the determined one or more oral surface characteristics as a function of position in the mouth of a surface to which the characteristics pertain. This can be understood as providing a spatial map of the derived oral surface characteristics. The map may be generated based on relating the derived oral surface characteristics to a position of the oral care device cleaning elements in the mouth as the data is acquired, and from this information an oral surface map may be generated. The oral surface map provides an intuitive way to display determined information about the state of oral hygiene of a user and can be easily communicated to a dental practitioner. The processing arrangement may be adapted to receive mouth positioning data corresponding the sampling period over which the sensor signal data is acquired, indicative of a position of the one or more cleaning elements within the mouth of the user during the sampling period. This allows position to be correlated or associated with the derived surface characteristics.

In some embodiments, the processing unit may be adapted to acquire the sensor signals over a sampling period, and wherein the processing unit is further adapted to compile a historical dataset of determined oral surface characteristics for a user based on storing the determined oral surface characteristics for each sampling period in a data store.

The sampling period may correspond to an operation session of the oral care device, e.g. a cleaning session. By storing a record of surface characteristics data from past uses of the oral care device, a history of surface characteristics can be observed and documented, such as tooth staining, tooth wear, tooth damage, presence of dental deposits or any other surface characteristics. Understanding where and how changes in tooth characteristics occur in the mouth can provide the user with feedback for preventing or reducing issues in the future.

In some embodiments, the processing unit may be further adapted to generate a sensory output for communicating to a user the determined oral surface characteristics. This provides the user real-time feedback that can be used to, for example, consult a dental practitioner where needed. The information determined by the oral care device may be communicated directly to the dental practitioner in further examples.

In accordance with one or more embodiments, the processing unit may be adapted to determine a baseline application force of the cleaning elements against the oral surface, e.g. as applied by a user. The determining of the oral surface characteristics may be further based on said baseline application force.

The application force is a baseline of the force exerted on the cleaning elements. The measured friction coefficient may depend upon this application force or contact pressure. By therefore taking into account this application force, the friction measurement can be corrected or calibrated according to the application force, improving accuracy.

The baseline force information may also be used in combination with a derived friction measure for determining one or more further surface characteristics. The relationship between friction and a given surface characteristics may depend upon the baseline application force. In this case, the baseline force measure may improve specificity of the derived further surface characteristic.

As will be described later, in some cases, the relationship between the friction coefficient and the baseline application force may be a logarithmic relation. However, it may alternatively be a different relationship, such as linear or polynomial relationship.

Examples in accordance with a further aspect of the invention provide an oral care device comprising: a set of flexible cleaning elements for engaging with oral surfaces; one or more sensors configured to generate one or more sensor signals indicative of force on and/or deflection of one or more cleaning elements; and a processing unit in accordance with any example or embodiment outlined above or described below, or in accordance with any claim of this application, the processing unit adapted to receive the one or more sensor signals.

The oral care device may be, by way of non-limiting example, a powered toothbrush or brushing mouthpiece device. The processing unit may be housed within the oral care device or may be separate from the oral care device.

In some embodiments, at least one cleaning element from the set of cleaning elements may be mounted to a surface of the oral care device by means of a resilient element, and wherein the resilient element is operatively coupled with at least one of the one or more sensors.

The resilient element may for example be a spring element.

By mounting at least one cleaning element to the brush head by means of a resilient element, a wide range of cleaning element position and/or motion information may be determined in an accurate manner. In some embodiments, the one or more sensors may comprise at least one of: a strain gauge; a capacitive gauge; and/or a Hall sensor.

In some embodiments, at least a subset of the elements may each comprise a pressure-responsive material adapted to generate an electrical signal (e.g. charge, current, voltage) dependent upon a deformation of the material, wherein each of the subset of cleaning elements forms at least part of one of the one or more sensors.

The pressure-responsive material may be an electroactive material such as a piezoelectric material, electroactive polymer or a conductive elastomer. By effectively placing the sensors in the location of a regular cleaning element, the generated (electrical) output signals provide a more reliable representation of the force or displacement experienced by the cleaning element. In some embodiments, each of the subset of cleaning elements may be combined with a fiber Bragg grating or a Fabry-Perot sensor.

In some embodiments, the oral care device may comprise at least one ring member arranged surrounding a base of at least one cleaning element, or a tuft of cleaning elements, and arranged so as to be mechanically engaged by the cleaning element or tuft of cleaning elements upon deflection thereof, and wherein the sensor signals are indicative of a force exerted on the ring element by the cleaning element or tuft of cleaning elements.

Where the ring member surrounds a tuft of cleaning elements, it preferably doubles as a tuft ring member, acting to hold a shape of the tuft.

In some embodiments, the ring member may be circumferentially segmented into a plurality of ring segments, and wherein the sensor signals are indicative of a force exerted on each segment of the ring member by the cleaning element or cleaning element tuft. By segmenting the ring member and detecting the force at each segment, directional information can be obtained.

The ring member may comprise a pressure-responsive material adapted to generate an electrical signal dependent upon a deformation of the material.

In the above embodiments, the sensor signals may be obtained from one or more ring members which are circumferentially continuous or which are circumferentially segmented, and each of which may surround a single cleaning element or a plurality of cleaning elements.

Determining cleaning element tuft position and/or motion information may allow for fewer sensors to be used.

The measured reaction force at the ring member provides a proxy for cleaning element force and/or deflection.

Examples in accordance with a further aspect of the invention provide a method, comprising: receiving one or more sensor signals indicative of force exerted on and/or deflection of one or more flexible cleaning elements of an oral care device, the one or more sensor signals covering a sampling period, obtaining cleaning element position and/or movement information for the sampling period based on the received one or more sensor signals; and determining one or more oral surface characteristics based on the cleaning element position and/or motion information, the one or more oral surface characteristics including a measure of surface friction.

Examples in accordance with a further aspect of the invention provide a computer program product comprising code means configured, when executed on a processor, to cause the processor to perform a method in accordance with any example or embodiment outlined above or described below, or in accordance with any claim of this application.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates experimental results indicative of friction force as a function of the number of cleaning element passes over a pellicle-stain surface;
Fig. 2 schematically illustrates variation in cleaning element position as a function of time during a cleaning session;
Fig. 3 schematically illustrates variation in angular position of a cleaning element due to surface friction change as a function of time during a cleaning session;
Fig. 4 illustrates variation in cleaning element position for a smooth surface compared with a rough surface;
Fig. 5 illustrates variation in cleaning element orientational pose for a smooth surface compared with a clean surface;
Fig. 6 illustrates determination of a cleaning element pose position in a reference coordinate system;
Fig. 7 illustrates an example strain gauge arrangement for measuring force on a cleaning element;
Fig. 8 illustrates use of cleaning elements comprising pressure-responsive material for sensing deflection;
Figs. 9-12 illustrate use of sensing ring members for sensing force exerted on cleaning elements;
Fig. 13 illustrates an oral care device in accordance with one or more embodiments; and
Fig. 14 illustrates an example method in accordance with one or more embodiments.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a means for determining oral surface characteristics based on acquiring force and/or deflection data pertaining to protruding cleaning elements of an oral care device, and deriving from the force and/or deflection data position or movement information for the cleaning elements. From the position or movement data, acquired over a time period, one or more surface characteristics such as friction can be determined. The surface characteristics may include, by way of example, tooth surface deposits such as tooth biofilm coatings, tooth staining, tooth wear or tooth damage.

It has been realized by the inventors that tooth surface deposits such as staining or biofilm coatings lead to changes in surface properties such as smoothness or stickiness of the tooth surface. Physically, these changes lead to modification of the friction coefficient of the tooth surface, as will be explained further below.

Embodiments of the present invention are based on detection of the position or motion dynamics of cleaning elements of an oral cleaning device (such as bristles) during use, or of forces on the cleaning elements during use. The inventors have established through experimentation that, when brushing a tooth with modified surface characteristics (such as a stained tooth or a tooth covered with stain pellicle), the cleaning element motion data exhibits detectable differences in the position, orientation (e.g. angular pose) and the dynamic response (i.e. response to applied forces) compared with brushing a clean surface.

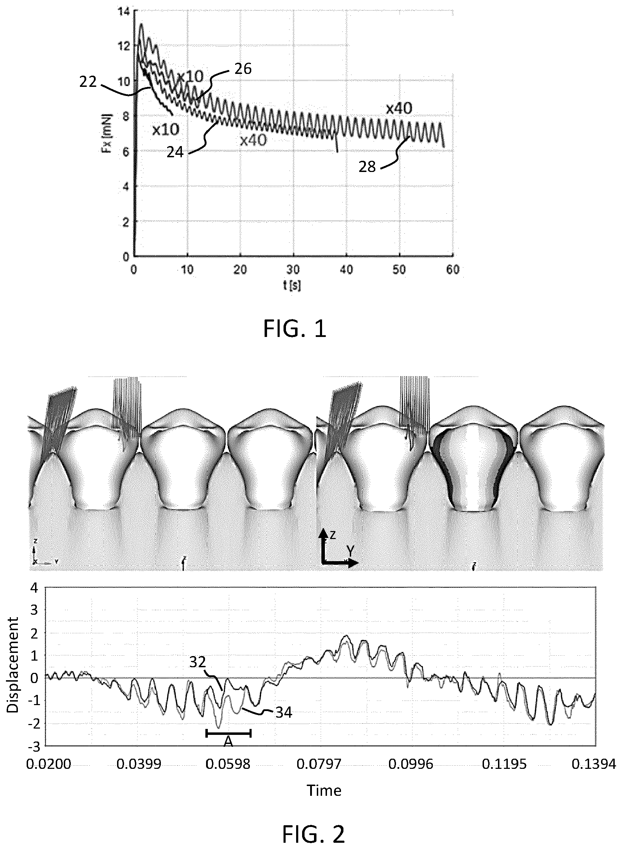

By way of further background, reference is made to Fig. 1, which represents results from experimentation performed by the inventors.

Stain removal and surface friction was assessed using a rotary tribometer setup in which a nylon bristle or nylon tuft (meaning a bundle of bristles) was slid against a human saliva-black tea pellicle-stain coated polymer. The friction and normal forces on the bristle or tuft were measured throughout, and a coefficient of friction derived. The coefficient of friction between an object and a surface can be understood as the ratio of the frictional force between the object and the surface, to the normal force. This can be derived from measuring the friction force experienced when moving the object across the surface and the normal force between the object and the surface.

From the experiments, it was found that the friction coefficient for a nylon bristle slid against the stained surface decreased from about 0.65 to 0.35 with increasing number of passes of the bristle against the stained surface, indicating gradual stain removal. In particular, each bristle pass performs a degree of cleaning of the surface. For a fully cleaned surface, a friction coefficient of 0.1-0.2 was measured. Thus, the stained surface exhibited a friction coefficient up to six times greater than that of the clean surface. It follows that stain removal may be can be detected by monitoring the friction coefficient as a function of the number of bristle passes, or as a function of time for a bristle oscillating at a uniform frequency.

The results of the above-described experiment are shown in Fig. 1. The graph shows friction or tangential force (y-axis) as a function of time (x-axis). The graph shows results for each of four trials, in each of which a bristle was passed multiple times along one of a plurality of concentric ring tracks of different respective lengths. Line 22 represents a friction signal from ten bristle passes along a ring track with radius of 11 mm, line 24 a friction signal of 40 bristle passes along a ring track with a radius of 15 mm, line 26 a friction signal of ten bristle passes along a ring track with radius 19 mm, and line 28 a friction signal of 40 bristle passes along a ring track with a radius of 23 mm. The test was performed on an *in vitro* tooth model surface which had been stained in advance with a saliva and black tea mixture.

For each of the different trials, it can be seen that with increasing numbers of passes of the bristle over the respective ring track, the friction force (y-axis) decreases (with normal force remaining constant). This therefore demonstrates that coefficient of friction of the tooth surface can be expected to decline as a function of declining staining level, and therefore coefficient of friction can provide a proxy measure of staining or cleanliness, or the presence of any other kind of surface coating which might affect smoothness of the surface.

Furthermore, experiments have also been performed by the inventors to demonstrate the relationship between position and/or movement patterns of bristles relative to a surface over which they are being rubbed, and the friction level of the surface. For example, this is illustrated in Fig. 2 which shows displacement in the Y direction (tangential direction, parallel to the tooth row) for a bristle being driven in an oscillatory motion pattern over the surface of a tooth. The graph shows Y displacement (y-axis) as a function of time (x-axis) for bristles being passed across a first surface (line 32) and a second surface (line 34). As can be seen, the amplitude of oscillation in the y-axis varies depending upon the changed position of the bristle across the tooth surface during brushing.

The first surface (line 32) has a higher friction indicative of greater staining, while the second surface (line 34) represents a low-friction, lower surface-stain region on the tooth. Displacement differences of approximately 1 mm between the two traces are visible during the time interval 0.05-0.07s. This time interval is labelled 'A' in Fig. 2. In addition to the displacement differences, there is also a visible stick-slip motion signature in the signal trace 32, characterized by a short plateau of near constant displacement (at around 0.06s), indicative of the bristle sticking, followed by sudden change in displacement (characterized by an overshoot displacement, or 'slip').

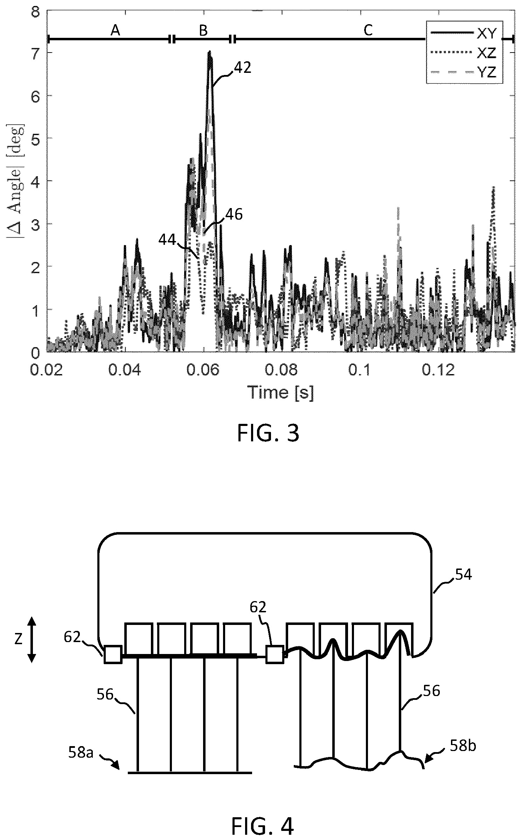

In addition to the motion pattern of a bristle varying as a function of the friction level of the surface it engages, also the average angular pose (or orientation) of a bristle relative to a reference vertical axis has been shown to vary depending upon the surface with which the bristle is engaging. This is illustrated for example in Fig. 3 which shows pose angle (y-axis; degrees) of a simulated bristle in each of an XY, XZ and YZ plane as a function of time (x-axis). The solid line 42 is angular pose in the XY plane, the dotted line 44 is angular pose in the XZ plane, and the dashed line 46 is the angular pose in the YZ plane. In the illustrated simulation, during time periods A and C, the bristle was applied to a clean tooth surface, and in time period B, the bristle was applied to a stained tooth surface. There is a clear change in the angular pose (orientation) of the bristle (of approximately 2-7 degrees) on a stained tooth surface compared to a clean tooth surface.

Although the above experiments were performed for cleaning elements in the form of bristles, it will be appreciated that the same principle applies also for other types of protruding cleaning element or member, such as elastomeric cleaning protrusions, or other types of cleaning filament, or tufts of cleaning elements.

There will now be outlined a number of embodiments of the invention, providing means for detecting oral surface characteristics, or proxies thereof, based on sensing of force or displacement of protruding cleaning members of an oral care device, and based on mapping said sensing data onto cleaning member position and/or motion data. From analysis of patterns in the position and/or motion data over a measurement or sampling period, surface characteristics, including a measure of surface friction, can be derived. In some embodiments, secondary surface characteristics may be derived based on the determined measure of surface friction.

According to at least a first aspect of the invention, there is provided a processing unit for an oral care device, adapted to: receive one or more sensor signals indicative of force on, and/or deflection of, one or more flexible cleaning elements of the oral care device; obtain position and/or motion information of the one or more flexible cleaning elements based on the received one or more sensor signals; and determine one or more oral surface characteristics based on the cleaning element position and/or motion information, the one or more oral surface characteristics including a measure of surface friction.

A data output may be generated indicative of the determined one or more oral surface characteristics.

The processing unit comprises one or more processors for performing the steps outlined above. The processing unit may further comprise an input/output or communication module operatively coupled with the one or more processors for receiving the one or more sensor signals, and optionally outputting a data output representative of the derived oral surface characteristics.

In some examples, the processing unit may be located in a housing of the oral care device. In some examples, the processing unit may be separated from the oral care device, e.g. embodied in a personal computing device communicatively coupled with sensing apparatus comprised by the oral care device.

There are different options for deriving the surface characteristics data, both in terms of the processing and analysis of the sensing data (software side) and in terms of the sensing apparatus utilized to acquire the sensing data (hardware side). The different options in both areas will now be outlined. It is to be understood that the various software-side options may be combined with any of the various hardware-side options. Furthermore, in one aspect of the invention, the software-side of the invention can be provided alone, e.g. embodied in a processing unit, or provided as computer-implemented method.

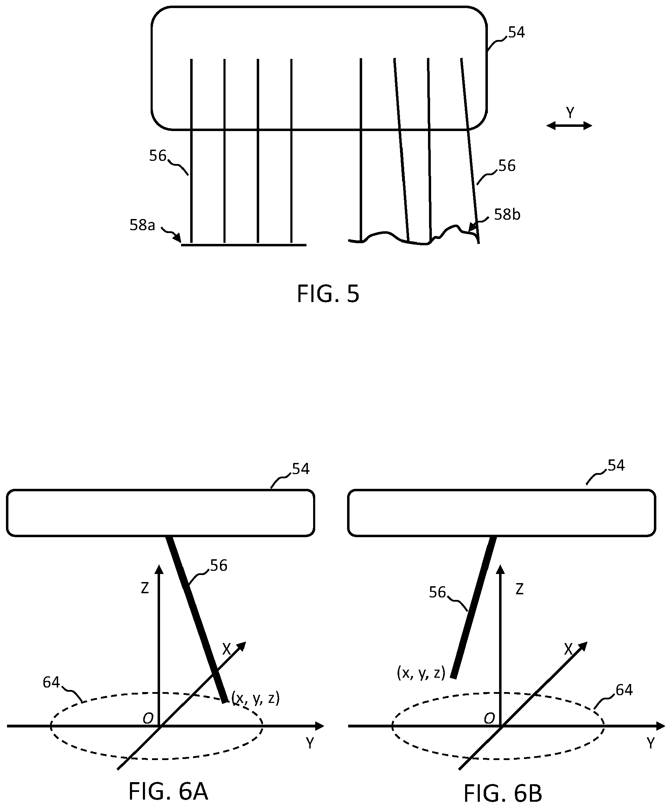

As noted above, average cleaning element pose position has been shown to vary depending upon the surface friction of the tooth. Thus, in one or more embodiments, the received sensor data may be processed to obtain information indicative of a pose position of one or a plurality of the cleaning elements over a measurement or sampling period covered by the sensor data. This is illustrated schematically in Fig. 4 and Fig. 5 which show an example oral care unit comprising a support body 54, e.g. a head of a toothbrush, from which outwardly extend a plurality of protruding cleaning elements or members 56, e.g. bristles. Fig. 4 illustrates how cleaning element distal tip position or displacement relative to the support body may differ for a smooth surface 58a compared to a surface 58b with higher surface friction. In particular, for a set of cleaning elements 56, the variation in pose positions between cleaning elements of the set is greater for a higher-friction surface than for a lower friction surface. Furthermore, the average displacement of any one cleaning element pose position from a natural rest position during a measurement period is greater for a higher friction surface.

The determined cleaning element pose position may include an angular position of the cleaning element or an orientation of the cleaning element, as illustrated in Fig. 5. The cleaning element pose position may further include a vertical displacement, i.e. in the z-direction, as illustrated in Fig. 4. The cleaning element position may be represented in terms of a displacement of a distal tip of the cleaning element 56 relative to a reference neutral point (an origin point, *O*), where the reference neutral point may for example be a point at which the cleaning element tip rests when no external force is applied to the cleaning element. This is illustrated schematically in Fig. 6 which shows a reference co-ordinate system, having origin point *O,* and relative to which cleaning element position may be determined. Thus, determining of the cleaning element position may mean determining an estimated position, displacement or angle of a distal tip of the cleaning element relative to a reference co-ordinate system, e.g. relative to a reference origin point within this co-ordinate system.

This is illustrated schematically in Fig. 6A and Fig. 6B, each of which shows a single cleaning element 56 which is deflected from a neutral resting position, e.g. in a direction normal to the support body 54 surface. The position of the cleaning element may be represented in terms of an estimated position of the distal tip of the cleaning element within the reference co-ordinate system X, Y, Z, the co-ordinate system having an origin, *O.* This results in a position co-ordinate for the distal tip (x, y, z). The cleaning element may have a maximum displacement. Fig. 6A and 6B each schematically illustrate an annular loop 64 in the X-Y plane illustrating maximum displacement of the cleaning element in this X-Y plane.

The cleaning element 56 position is shown in Fig. 6 as being represented in 3D Cartesian co-ordinates. It may instead be represented in polar or cylindrical co-ordinates. In this case, it may be represented in terms of a radial distance, r, of the cleaning element tip from the origin, in combination with a polar angle and azimuthal angle of the element tip relative to the x-axis and z-axis respectively.

Fig. 6 shows displacement of a single cleaning element. However, in practice, the position of each of a plurality of cleaning elements may be determined, for example based on force, displacement and/or elastic deformation sensor measurements for each of the plurality of cleaning elements. For example, Fig. 4 shows use of a strain gauge device 62 for measuring force, displacement or elastic deformation of each cleaning element 56 of a plurality of cleaning elements. Fig. 4 shows z-direction force, displacement and/or elastic deformation being measured. However, force, displacement and/or elastic deformation in two or three dimensions may be measured in further examples.

In some examples, the one or more oral surface characteristics may be derived from the position data derived for the plurality of cleaning elements. In some examples, the oral surface characteristics may be derived based on a measure of variance in the positions or average positions of the plurality of cleaning elements over a measurement period. The measurement period may for example be a regular oral cleaning session, or it may be a plurality of brushing sessions. Where it is a plurality of brushing sessions, the position data may be averaged over the plurality of sessions.

For example, if there is high variation between the positions of the cleaning elements at a given moment in time, or over the course of a time window, this may indicate that the surface has a higher friction level. In other examples, the oral surface characteristics may be derived based on the absolute positions of one or a plurality of the cleaning elements, at a single moment, or a time average of the position over a time window.

In some examples, a lookup table or database may be used which associates measured cleaning element position (for a single cleaning element, or a plurality of cleaning elements) with values for one or more surface characteristics. This may be a time-average position over a time period for the single cleaning element or plurality of cleaning elements in some examples.

According to one or more examples, the acquired cleaning element position or motion data may be compared with baseline or reference data, e.g. acquired a professional cleaning or whitening treatment. This allows for drift in the motion patterns exhibited by the cleaning elements, indicative of changing surface characteristics, to be detected.

In the above examples, the sensor data is used to derive cleaning element position information. Additionally or alternatively, the sensor data can be used to derive cleaning element motion information. The motion information may comprise data indicative of cleaning element position or deflection as a function of time, or of bristle force as a function of time. The motion information may comprise data indicative of bristle kinematics or dynamics.

As discussed above, the movement pattern exhibited by a cleaning element has been shown to vary depending upon the friction level of the surface with which it is engaged. It may exhibit different characteristic motion pattern signatures when applied to surfaces having different characteristics. Thus according to one or more embodiments, the processing unit may be adapted to derive from received sensor data a measure of a position of each of a plurality of cleaning elements, or just a single cleaning element, as a function of time over a sampling or measurement period for which sensor signals are received. This may be referred to as motion data for the one or more cleaning elements for the sampling period. The motion data may additionally or alternatively comprise force as a function of time, acceleration as a function of time, angular pose as a function of time, or any other metric representative of spatial position or motion as a function of time over the measurement period.

The motion data may be processed or analyzed to determine one or more surface characteristics, where the surface characteristics include at least a measure of surface friction. In at least one set of embodiments, the motion data can be processed to identify or extract one or more pre-defined motion signatures or motion patterns within the motion data, the motion signature or motion pattern being associated with a particular oral surface characteristic. For example, a high friction surface may exhibit a stick-slip motion pattern which is identifiable as a sudden sharp increase in motion speed from static to non-static. In other words, the cleaning element exhibits a pattern of sticking to the surface (statically), followed by a transient motion across as the oral surface as the cleaning element breaks free from the surface (a stick-slip motion). By contrast, a low friction surface may exhibit a smooth, continuous motion pattern.

Additionally or alternatively, the motion data may be processed to determine one or more properties of the motion signal, for example an amplitude of an oscillatory motion of the cleaning element.

In any of the above examples, the acquired cleaning element position and/or motion data is used to derive surface characteristics information, including a measure of surface friction. This may for example be a surface coefficient of friction. The mapping from the position and/or motion data to a measure of surface friction may be performed using a lookup table or database. In further examples, it may be achieved using a machine learning algorithm such as a neural network which has been trained so as to receive as an input position and/or motion data for one or a plurality of cleaning elements over a sampling period, and to generate as an output a measure indicative of surface friction of a surface with which the cleaning elements were engaged.

There are different means for obtaining the sensor data indicative of force on, and/or deflection of, the one or more cleaning elements.

According to an aspect of the invention, there is provided an oral care device, comprising: a set of flexible cleaning elements for engaging with oral surfaces; one or more sensors configured to generate one or more sensor signals indicative of force on and/or deflection of one or more cleaning elements, the one or more sensor signals covering a sampling period; and a processing unit in accordance with any example or embodiment outlined above or described below, or in accordance with any claim of this application.

The one or more cleaning elements may for example be bristles, for example nylon bristles. The one or more cleaning elements may be elastomeric cleaning members, for example cleaning rods or probes. The elastomeric material may be a rubber material or a silicone material for example.

In accordance with one or more embodiments, the sensor signals may be obtained using one or more sensors which each comprise a strain gauge, a capacitive gauge and/or a Hall sensor.

According to one or more embodiments, at least one cleaning element from the set of cleaning elements may be mounted to a surface of the oral care device by means of a resilient element, and wherein the resilient element is operatively coupled with at least one of the one or more sensors. The resilient element may for example take the form of a spring element. The spring element may comprise a helical spring element, a leaf spring element, or a loose rubber spring element for instance.

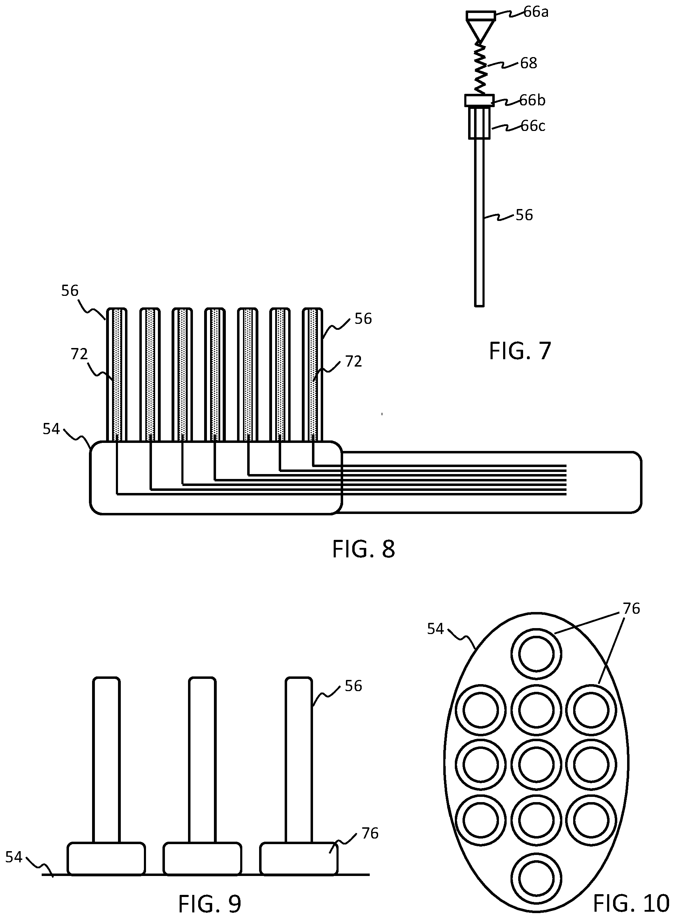

One example is illustrated in Fig. 7 which shows an example cleaning element fitted to an example strain gauge arrangement. A proximal end of the cleaning element 56 is coupled to a first strain gauge sensor 66a via a spring element 68. A proximal end of the cleaning element 56 is also directly coupled to a second strain gauge sensor 66b. A further pair of strain gauge sensors 66c is also provided at either side of the proximal base of the cleaning element. By providing strain gauge sensors at three locations, force exerted on the cleaning element at a distal end can be sensed in three dimensions.

Instead of strain gauge sensors, capacitive (plate) sensors, Hall sensors, a fiber Bragg grating sensor and/or a Fabry-Perot sensor can be used.

The sensors 66a, 66b, 66c and the resilient element 68 may be received within a space or cavity recessed into the surface of the oral cleaning device.

During use of the oral care device for an oral care function, e.g. during tooth brushing, the resilient element will deflect in the space in which it is located to couple the force exerted on the distal end of the bristle to the sensor 66a.

In addition to or instead of the sensing elements outlined above, according to one or more embodiments, the set of cleaning elements may comprise at least a subset of cleaning elements which each comprise a pressure-responsive material adapted to generate an electrical signal dependent upon a deformation of the material. Each cleaning element having the pressure responsive material may form at least part of one of the one or more sensors. The pressure responsive material may comprise an electroactive polymer and/or a piezoelectric material.

An example is illustrated schematically in Fig. 8 which shows a support unit 54 from which outwardly extends a plurality of flexible cleaning elements or members 56. For example, the support unit may be a head of a toothbrush, and the cleaning elements may be bristles or elastomeric cleaning members. Each cleaning element comprises an axial core 72 formed of a pressure-responsive material such an electroactive polymer. In the illustrated example, the axial core is surrounded by an insulating sheath to protect the user from electrical currents. However, depending upon the pressure-responsive material used, this may not be essential. Furthermore, in the illustrated example, the pressure-responsive core extends along the full longitudinal length of the cleaning element. However, this is also not essential. The pressure responsive material may extend along only a portion of the cleaning element length.

An electrical connection track such as a wire extends from a proximal base of each cleaning element and is electrically connected to the axial core of each cleaning element. During use, deflection of the cleaning elements 56 as they are passed over oral surfaces induces a current or voltage to be generated by the pressure-responsive material 72 which varies as a function of cleaning element deflection. This results in a separate sensing signal being generated by each cleaning element core which is communicated to a processing unit. The processing unit may for example be located in a housing of the oral care device, and a wired connection may be provided between the cleaning elements and the processing unit. Alternatively, the sensing signals may be coupled to a communication module adapted to transmit the sensing signals wirelessly to a remote processing unit.

By integrating the pressure-responsive material in the cleaning element, the cleaning element provides a dual function of both sensing and cleaning.

In accordance with a further one or more embodiments, the oral care device may comprise at least one ring member arranged surrounding a base of at least one cleaning element, or a tuft of cleaning elements. The ring member is arranged so as to be mechanically engaged by the cleaning element or tuft of cleaning elements upon deflection thereof. The sensor signals may be indicative of a force exerted on the ring element by the cleaning element or tuft of cleaning elements. The ring member may incorporate pressure sensing means. The ring member may in some example be operable to sense shear or tangential forces as well as normal forces.

Fig. 9 and Fig. 10 schematically illustrate an example in accordance with this set of embodiments. Each of a plurality of cleaning elements 56 is surrounded at a proximal base with a ring member 76. Alternatively, each ring member may surround a base of a tuft of cleaning elements. Fig. 10 schematically illustrates a plan view of an example support unit 54, such as a head of a toothbrush, comprising a plurality of ring members 76 surrounding tufts of cleaning elements.

Each ring member may comprise a pressure-responsive material adapted to generate an electrical signal dependent upon a deformation of the material. The pressure responsive material may for example be an electroactive polymer, a pressure-responsive elastomer, a ferroelectric-dielectric material, and/or a piezoelectric material. Alternatively, sensing can be achieved by providing sensing elements disposed on an interior surface of the ring member, e.g. a strain gauge deposited and/or printed on an interior surface of the ring member.

During use, deflection of the cleaning element 56 causes a force or pressure to be exerted against the ring member 76 which can be sensed to provide a sensing signal indicative of the deflection or force on the cleaning element. Normal force may optionally also be sensed by including a base to the ring element capable of sensing normal forces. In this way, the ring member functions as a 3D dynamometer or a 3D force/moment sensor.

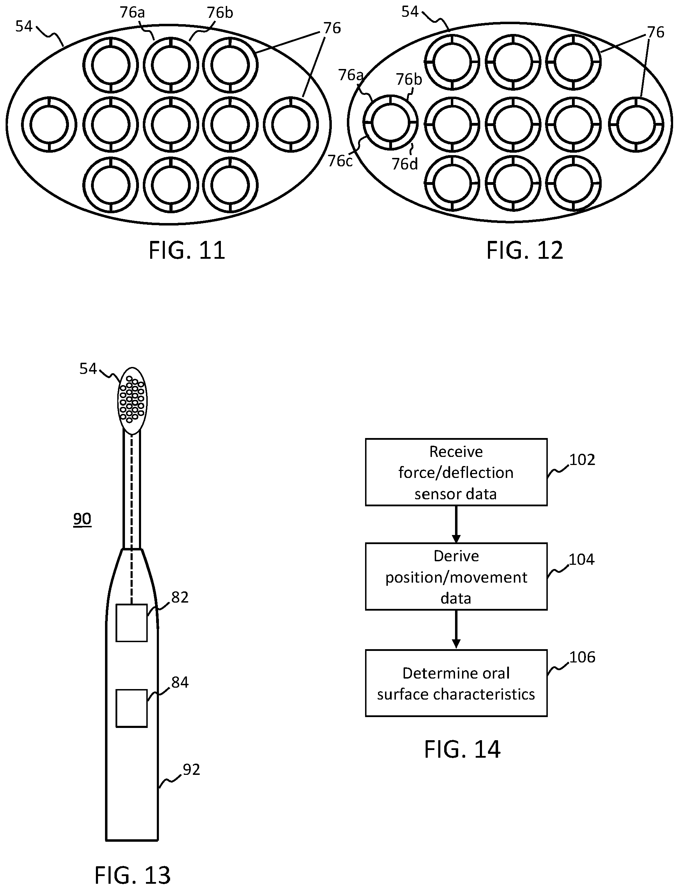

According to some embodiments, at least a subset of the ring members may be circumferentially segmented into a plurality of ring segments. The sensor signals may be indicative of a force exerted on each segment of the ring member by the cleaning element or cleaning element tuft. Fig. 11 shows one example in which the ring members are segmented into two segments 76a, 76b. Fig. 12 shows a further example in which the ring members are segmented in four segments 76a, 76b, 76c, 76d. In further examples, any number of segments can be provided. By segmenting the ring members, (higher resolution) force direction information or motion direction information of one or a set of cleaning elements (e.g. a tuft) can be obtained.

Above have been described examples of position and/or motion data which may be acquired using the sensor data, and example hardware options for obtaining the sensor data. There will now be described various options relating to the deriving of the one or more oral surface characteristics.

The oral surface characteristics may include by way of non-limiting example, tooth surface characteristics, gum surface characteristics, and characteristics of oral prosthetics and implants.

The derived one or more oral surface characteristics preferably include at least a measure of surface friction. As noted above, it has been found that tooth staining level is correlated with surface friction.

The one or more oral surface characteristics may include, by way of non-limiting example a tooth staining level, a tooth staining color, a measure of physical wear to the tooth surface (e.g. surface erosion), presence and/or category of bacterial biofilms, presence of dental tartar, presence of lesions in the gums, or presence and status of orthodontic structures such as prosthetics or implants.

The derived one or more oral surface characteristics may include a detection of presence or absence of a deposit on a tooth surface, e.g. a biofilm deposit, dental tartar or dental plaque. The derived surface characteristic in this case could be a binary metric indicative of presence or absence of the relevant coating. This can be determined from the friction measure or directly from the cleaning element position and/or motion patterns over a sampling period.

In some examples, the processing arrangement may be further adapted to receive positioning data indicative of a position of the one or more cleaning elements within the mouth of the user. The determining of the one or more surface characteristics may in some examples further be based on the mouth positioning data.

Determining the one or more oral surface characteristics may comprise accessing a database comprising data which relates cleaning element position and/or motion information to oral surface characteristics. In some examples, the database may associate pre-defined motion signatures or motion patterns with one or more surface characteristics. The system may be adapted to process the sensor data to detect and/or extract the pre-defined motion signatures or patterns (if present), and use these to determine the surface characteristics.

In some embodiments, determining of the one or more surface characteristics may be achieved using a machine learning algorithm such as a neural network which has been trained so as to receive as an input position and/or motion data for one or a plurality of cleaning elements over a sampling period, and to generate as an output a measure indicative of at least one oral surface characteristic of a surface with which the cleaning elements were engaged.

As noted, in an advantageous set of embodiments, the method comprises determining a measure indicative of staining of a tooth surface. The derived oral surface characteristics may include a measure indicative of tooth staining color. The derived oral surface characteristics may include a measure indicative of tooth staining level.

Based on experimental data, it has been found by the inventors the degree of tooth staining can be accurately characterized means of a measure of the surface friction coefficient. Thus, embodiments may include a lookup table, database or other mapping means permitting mapping between derived friction coefficient and a measure of tooth staining, for example a Lobene stain index.

Extrinsic dental stains can have different causes ranging from poor oral hygiene to consumption of certain substances such as tobacco or polyphenol chromogen-rich beverages and drinks (e.g. coffee, tea). Based on the underlying cause, e.g. the type of substance responsible for the stain, the chemical composition of a stain can change. The chemical composition of the staining also affects the surface topography, roughness, hardness and other surface properties. Thus, according to one or more embodiments, the type of stain, e.g. the color of the stain or the cause of the stain, may be determined based on the derived friction coefficient or based on cleaning element position and/or motion data. This may be achieved for example by using predefined thresholds based on an experimental database and/or machine learning based methods.

According to one or more embodiments, the one or more derived surface characteristics may include detection of structural, geometrical, topographical or morphological properties of surfaces. For example, a change to the structural form or shape or topography of a surface may indicate that damage has occurred, e.g. a chip or scratch. This information can be obtained from analysis of the position and/or motion data. For example, the pattern of cleaning element motion when cleaning a particular tooth

In accordance with one or more embodiments, the processing unit may be adapted to acquire the sensor signals over a sampling period, and wherein the processing unit is further adapted to compile a historical dataset of determined oral surface characteristics based on storing the determined oral surface characteristics for each sampling period in a data store. For example, the historical dataset may be stored in a local or remote datastore. For example, it may be stored in a cloud-based datastore with which the processing unit communicates via an Internet connection.

Using the historical dataset, the determined surface characteristics may be monitored over time to detect changes or trends. Derived values may be averaged over multiple brushing sessions covering a pre-defined time period, e.g. a week, and compared to a reference level or baseline. The reference level may be a level acquired immediately after a cleaning treatment, e.g. after a whitening treatment or a professional cleaning. The reference level may a population-based level derived from pooled data acquired for a plurality of users, or of a general population. Any large change in the surface properties may be reported to the user.

In some examples, the processing arrangement may be further adapted to receive device positioning data indicative of a position of the one or more cleaning elements within the mouth of the user. The processing arrangement may further be adapted to generate a digital representation of the determined one or more oral surface characteristics as a function of position in the mouth. This may be known as a map of the surface characteristics. The map may simply comprise a data representation of the derived surface characteristics and the mouth location to which the characteristics correspond. Additionally or alternatively, a visual representation of the map may be generated, for example a visual depiction of the mouth, with a visual representation of one or more surface characteristics overlaid thereupon, e.g. using a color-based representation or using textual representation. The device positioning data may be obtained using one or more location sensors, e.g. an inertial measurement unit (IMU) integrated in the oral care device to detect pose of the oral care device. By way of example, suitable means for implementing position tracking of the oral care device are outlined in the document US 2020/069042.

In one set of examples, the digital mapping can be used for mapping staining of teeth in the mouth. The resulting map may be referred to as a digital stain mouth map. This advantageously enables triaging by a clinician, to determine suitable procedures for cleaning, whitening or for improving gum health. A risk stratification can be performed based on the results, e.g. rough teeth are correlated with greater bacteria growth.

In addition to use by clinicians, a derived map enables a user to assess their own cleaning technique, for instance by reviewing a staining map retrospectively after a cleaning session, or by looking at a staining map in real time as they perform a cleaning session. As noted above, the measured friction coefficient can be used as a proxy measure for stain level or color, and therefore permit tracking of stain removal.

In accordance with one or more embodiments, the processing unit may be further adapted to generate a sensory output for communicating to a user the determined oral surface characteristics. For example, a visual representation of obtained oral surface characteristics information may be generated and output to a display device. The display device may be an auxiliary display device. It may be provided as part of a system with the processing unit and/or the oral care device.

In accordance with one or more embodiments, the processing unit may be adapted to determine a baseline application force of the cleaning elements against the oral surface by a user. The determining of the oral surface characteristics may be further based on said baseline application force.

The application force is a baseline of the normal force or load exerted on the cleaning elements by the user applying them to the oral surface(s). The measured friction coefficient depends upon this application force. In some cases, the relationship between the two may be a logarithmic one. By therefore taking into account this application force, the friction measurement can be corrected or calibrated according to the application force, improving accuracy.

The calibration may be performed based on a pre-determined relationship between friction coefficient and normal load/pressure. From such a relation, it is possible to determine the pressure dependency and correct the measured friction force or friction coefficient accordingly. This thereby makes the friction measurement more specific and robust to different baseline normal force levels.

As noted above, examples in accordance with an aspect of the invention provide an oral care device.

Fig. 13 shows an example oral care device 90 in the form of a toothbrush. The toothbrush comprises a handle portion 92 having a housing containing a processing unit 82 in accordance with any embodiment disclosed in this disclosure or in accordance with any claim of this application. The device comprises a head portion detachably coupled to the handle portion. The head portion comprises a brush head section at a distal end forming a support body 54, and comprising a plurality of cleaning elements in the form of bristles outwardly extending from a surface thereof. The brush head section comprises one or more sensors configured to generate one or more sensor signals indicative of force on and/or deflection of one or more cleaning elements.

The device 90 preferably further comprises a motor 84 and a drive train mechanism for applying oscillatory force to the brush head section to induce oscillatory motion of the cleaning elements. The device 90 preferably further comprises a rechargeable battery for powering the motor.

Although Fig. 13 shows an oral care device in the form of a toothbrush, other types of oral care device may be provided in accordance with further embodiments. By way of non-limiting example, the oral care device may comprise: a brushing mouthpiece device, an oral irrigator device, or a powered flossing device.

Examples in accordance with a further aspect of the invention provide a system comprising a processing arrangement in accordance with any example or embodiment outlined above or described below, or in accordance with any claim of this application; and further comprising an oral care device comprising a set of flexible cleaning elements for engaging with oral surfaces; and one or more sensors configured to generate one or more sensor signals indicative of force on and/or deflection of the one or more cleaning elements.

Examples in accordance with a further aspect of the invention provide a method. Fig. 14 is a block diagram of an example method. The method comprises receiving 102 one or more sensor signals indicative of force on and/or deflection of one or more flexible cleaning elements of an oral care device; obtaining 104 cleaning element position and/or movement information based on the received one or more sensor signals; and determining 106 one or more oral surface characteristics based on the cleaning element position and/or motion information, the one or more oral surface characteristics including a measure of surface friction. The method may be a computer-implemented method.

As discussed above, the one or more oral surface characteristics may, by way of non-limiting example, include the presence of staining, surface wear, biological coating materials (e.g. dental tartar or biofilms) or non-biological materials (implants, crowns).

Examples in accordance with a further aspect of the invention also provide a computer program product comprising code means configured, when executed on a processor, to cause the processor to perform a method in accordance with any example or embodiment outlined above or described below, or in accordance with any claim of this application.

As discussed above, embodiments make use of one or more processors to perform the data processing. The one or more processors can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A processing unit (82) for an oral care device (90), adapted to:
receive one or more sensor signals indicative of force on, and/or deflection of, one or more flexible cleaning elements (56) of the oral care device over a sampling period;
obtain position and/or motion information of the one or more flexible cleaning elements for the sampling period based on the received one or more sensor signals; and
determine one or more oral surface characteristics based on the cleaning element position and/or motion information, the one or more oral surface characteristics including a measure of surface friction.

2. The processing unit of claim 1, wherein the determining the one or more surface characteristics comprises detecting, based on the position and/or motion information, a stick-slip condition of the oral surface.

3. The processing unit of claim 1 or 2, wherein the one or more oral surface characteristics include one or more of: a tooth staining level; a tooth staining color; a measure of physical wear to a tooth surface; presence of bacterial biofilms; presence of dental tartar; presence of gum lesions; and presence of prosthetic structures.

4. The processing unit of any of claims 1-3, wherein determining the one or more oral surface characteristics comprises:
accessing a database comprising data which relates cleaning element position and/or motion information to oral surface characteristics; and/or
using one or more machine learning algorithms, trained to receive an input comprising position and/or motion data for the one or more cleaning elements, and to generate an output comprising a measure indicative of one or more oral surface characteristics.

5. The processing unit of any of claims 1-4, further adapted to generate a data representation of the determined one or more oral surface characteristics as a function of position in the mouth.

6. The processing unit of any of claims 1-5, wherein the processing unit is further adapted to compile a historical dataset of determined oral surface characteristics for a user based on storing the determined oral surface characteristics for each sampling period in a data store.

7. The processing unit of any of claims 1-6, wherein the processing unit is further adapted to generate a sensory output for communicating to a user the determined oral surface characteristics.

8. An oral care device (90), comprising:
a set of flexible cleaning elements (56) for engaging with oral surfaces;
one or more sensors configured to generate one or more sensor signals indicative of force on and/or deflection of the one or more cleaning elements; and
a processing unit (82) as claimed in any of claims 1-7, adapted to receive the one or more sensor signals.

9. The oral care device of claim 8, wherein at least one of the set of cleaning elements is mounted to a surface of the oral care device by means of a resilient element, and wherein the resilient element is operatively coupled with at least one of the one or more sensors.

10. The oral care device of claim 8 or 9, wherein the set of cleaning elements comprises at least a subset of cleaning elements each comprising a pressure-responsive material adapted to generate an electrical signal dependent upon a deformation of the material, wherein each cleaning element of the subset forms at least part of one of the one or more sensors.

11. The oral care device of any of claims 8-10, wherein the oral care device comprises at least one ring member arranged surrounding a base of at least one cleaning element, or a tuft of cleaning elements, and arranged so as to be mechanically engaged by the cleaning element or tuft of cleaning elements upon deflection thereof, and wherein the sensor signals are indicative of a force exerted on the ring element by the cleaning element or tuft of cleaning elements.

12. The oral care device of claim 11, wherein the ring member is circumferentially segmented into a plurality of ring segments, and wherein the one or more sensor signals are indicative of a force or pressure exerted on each segment of the ring member by the cleaning element or cleaning element tuft.

13. The oral care device of claim 11 or 12, wherein the ring member comprises a pressure-responsive material adapted to generate an electrical signal dependent upon a deformation of the material.

14. A method, comprising:
receiving (102) one or more sensor signals indicative of force on, and/or deflection of, one or more flexible cleaning elements of an oral care device over a sampling period,
obtaining (104) cleaning element position and/or movement information for the sampling period based on the received one or more sensor signals; and
determining (106) one or more oral surface characteristics based on the cleaning element position and/or motion information, the one or more oral surface characteristics including a measure of surface friction.

15. A computer program product comprising code means configured, when executed on a processor, to cause the processor to perform a method in accordance with claim 14.
